# EUROPEAN PATENT APPLICATION

(11) **EP 4 570 900 A1**
(43) Date of publication of application: **18.06.2025**
(21) Application number: 23851673.6
(22) Date of filing: 03.08.2023
(51) Int. Cl.: C12N 1/20, A61K 35/747, A61P 31/02, A61P 31/04, A61P 31/10, A61P 15/02, C12R 1/25

(54) **LACTIPLANTIBACILLUS PLANTARUM STRAIN AND USE THEREOF**

(30) Priority: 09.08.2022 CN 202210950834
(71) Applicant: Chengdu Brilliant Biopharmaceutical Co., Ltd., Chengdu, Sichuan 610200 (CN); Chengdu Brilliant Pharmaceutical Co., Ltd., Chengdu, Sichuan 610094 (CN)
(72) Inventor: HUANG, Haoxi, Chengdu, Sichuan 610200 (CN); CUI, Yangwen, Chengdu, Sichuan 610200 (CN); LIU, Jiali, Chengdu, Sichuan 610200 (CN); LIU, Wu, Chengdu, Sichuan 610200 (CN)
(74) Representative: Aera A/S
(86) International application number: PCT/CN2023/110924
(87) International publication number: WO 2024/032452

(57) **Abstract**

Provided is a *Lactiplantibacillus plantarum* 1-D1 strain and use thereof. The strain has genetic stability, no pathogenic/virulence genes, good safety, strong antibacterial ability against pathogenic bacteria, can reduce the pH in vagina, maintain a healthy vaginal acidic environment, and has outstanding adhesion ability to Hela cells, can adhere to vaginal epithelial cells, form a microecological barrier, and prevent pathogens from colonizing or competing for epithelial cell receptors. The strain can be used in the preparation of drugs, foods, and sanitary products for preventing and treating vaginitis.

## Description

### TECHNICAL FIELD

The present application belongs to the technical field of microorganisms, and particularly relates to a *Lactiplantibacillus plantarum* strain and use thereof.

### BACKGROUND

In the vagina of healthy women of childbearing age, *Lactobacillus* is the dominant flora, accounting for more than 70% of the vaginal flora. Studies have shown that *Lactobacillus* in the female reproductive tract inhibits pathogenic microorganisms mainly by producing lactic acid and bacteriocin.

Among the outpatients of obstetrics and gynecology in China, reproductive tract infections account for 40.2%-55.6%. There are at least 200 million cases of reproductive tract infection-related diseases, of which 100 million cases of recurrence, resulting in medical costs as high as more than 20 billion yuan per year. Surveys show that 99.3% of patients with symptomatic vaginal infections in gynecological outpatient clinics have vaginal microecological imbalance. In 2016, the Infection Collaboration Group of the Obstetrics and Gynecology Branch of the Chinese Medical Association proposed that the essence of vaginal infection is "vaginal microecological imbalance". If the vaginal microecology is in an abnormal state for a long time, the vagina's resistance to pathogenic microorganisms will be reduced, which is often an important cause of recurrent vaginal infections or secondary new infections.

Common vaginal infections include: bacterial vaginosis (BV), vulvovaginal candidiasis (VVC), trichomonas vaginitis (TV), aerobic vaginitis (AV), etc., all of which are accompanied by a reduction or disappearance of *Lactobacillus.* Conventional therapeutic regimens of vaginal infection are mainly "antibacterial". After treatment with antibiotics such as clindamycin, bacterial vaginal pathogenic bacteria are inhibited while the growth of *Lactobacillus* in the vagina is also inhibited. Although metronidazole does not inhibit the growth of *Lactobacillus* at the the dosage administered, it cannot promote the recovery of *Lactobacillus.* Studies have shown that the resistance rate of metronidazole is 63.8%, and the resistance rate of clindamycin is 24.1%~67%.

In addition, the recurrence rate and drug resistance rate after using antibiotics are very high. Antibiotics can only inhibit the planktonic *Gardnerella vaginalis* and temporarily control symptoms, but cannot completely eliminate the biofilm and the *Gardnerella vaginalis* in it. Once the treatment is stopped, the bacteria in the biofilm will revive, multiply, and spread again, leading to the recurrence of bacterial vaginosis. Biofilms can reduce the sensitivity of bacteria to antimicrobial drugs and enhance their resistance to antimicrobial drugs. Moreover, the resistance of pathogenic strains can be acquired through mobile genetic elements, which will cause the therapeutic effect of antibiotics to become weaker and even ineffective.

Research of Narisu et al. showed that vaginal *Lactobacillus* can interfere with the formation of the biofilm of *Gardnerella vaginalis.* At the present stage, the Infection Collaboration Group of the Obstetrics and Gynecology Branch of the Chinese Medical Association pointed out that vaginal microecological preparations can be used to restore a weakly acidic environment dominated by functional *Lactobacillus,* promote the balance of vaginal microecology and immune regulation, and reduce the recurrence of vaginal infections (Narisu, et al. The effects of Lactobacillus on gardnerella vaginalis biofilm [J]. Progress in Modern Obstetrics and Gynecology, 2015, 24(09): 641-645.). Currently, there are only two types of vaginal microecological preparations on the market in China. One is a commercial available product under the trade name "Yanhua" produced by Xi'an Zhenghao Biopharmaceutical Co., Ltd., which contains a *Streptococcus faecalis* strain, that is not a dominant strain in vagina, and the bacteria under this species are conditionally pathogenic. The other is a commercial available product under the trade name "Wanze Shuangqi" produced by Inner Mongolia Shuangqi Pharmaceutical Co., Ltd., which contains a *Lactobacillus* strain *- Lactobacillus delbrueckii.*

The vaginal microecological preparations currently on the market are far from meeting clinical needs, and there is still a lot of room for optimization. Therefore, if *Lactobacillus* strains with stronger probiotic ability can be developed for the prevention and/or treatment of vaginal infections, it would be of great application value.

### SUMMARY

The purpose of the present application is to provide a *Lactiplantibacillus plantarum* 1-D1 strain, which can inhibit the growth of pathogenic bacteria in the vagina, regulate the balance of vaginal microecology, have the effect of preventing and treating vaginitis, and can be used in the preparation of drugs, foods, and sanitary products for preventing and treating vaginitis.

In order to realize the objects of the present application, the present application provides the following technical solutions:
A *Lactiplantibacillus plantarum* 1-D1 strain, the accession number of the strain is: CCTCC NO: M 20221191.

Use of the *Lactiplantibacillus plantarum* 1-D1 strain provided by the present application in the preparation of antibacterial products.

Furthermore, the antibacterial products include but are not limited to bacterial inhibitors, feed additives and antibacterial peptides, etc.

Furthermore, the antibacterial product is a product that inhibits one or more bacteria of *Gardnerella, Staphylococcus aureus, Pseudomonas aeruginosa, Escherichia coli, Salmonella,* and *Shigella dysenteriae.*

Use of the *Lactiplantibacillus plantarum* 1-D1 strain provided by the present application in the preparation of products for preventing and/or treating vaginal infectious diseases.

The term "treatment" (also referred to as "treat" or "treating") refers to any administration of a therapeutic agent according to a therapeutic regimen that achieves a desired effect, i.e., partially or completely alleviates, improves, ameliorates, inhibits, delays the onset of, reduces the severity of, and/or reduces the incidence of one or more symptoms or features of a particular disease, disorder, and/or condition (e.g., improving the structure and function of vaginal flora, increasing the diversity of vaginal flora, reducing the abundance of inflammation-related flora, treating vaginitis). In some embodiments, the administration of a therapeutic agent according to a therapeutic regimen is associated with the achievement of a desired effect. Such treatment may be directed to a subject who does not exhibit the relevant disease, disorder, and/or condition, and/or to a subject who only exhibits early signs of the disease, disorder, and/or condition. Alternatively or additionally, such treatment may be directed to a subject who exhibits one or more identified signs of the relevant disease, disorder, and/or condition. In some embodiments, the treatment may be directed to a subject who has been diagnosed with the relevant disease, disorder, and/or condition. In some embodiments, the treatment may be directed to a subject who is known to have one or more susceptibility factors that are statistically correlated with an increased risk of developing the relevant disease, disorder, and/or condition.

The products include but are not limited to drugs, foods, health products, sanitary products, etc. When used in the products, the strain is an active strain or an inactive strain.

The term "drug" encompasses drugs used in human medicine and veterinary medicine for both human and animal use, as well as drugs for incorporation into animal feed (such as livestock feed and/or pet food). In addition, the term "drug" as used herein means any substance that provides therapeutic, preventive and/or beneficial effects. The term "drug" as used herein is not necessarily limited to substances requiring a Marketing Approval, but includes substances that can be used in cosmetics, health products, foods (including, for example, feed and beverages), probiotic cultures and dietary supplements.

The *Lactiplantibacillus plantarum* 1-D1 strain of the present application may be used as a beneficial ingredient to be added to fermented foods, immunity-enhancing health foods, female private care solid beverages and other foods, which can enhance human immunity, especially enhance the immunity of the female reproductive tract.

The vaginal infectious disease is caused by one or more of bacterial vaginal pathogenic bacterium, *vulvovaginal candidiasis* pathogenic bacterium, trichomonas vaginitis, and aerobic vaginitis.

Furthermore, the pathogenic bacterium of the aerobic vaginitis includes but is not limited to *Staphylococcus aureus, Escherichia coli, Pseudomonas aeruginosa, Shigella dysenteriae* or *Salmonella,* etc..

Furthermore, the bacterial vaginal pathogenic bacterium is *Gardnerella vaginalis.*

Furthermore, the product is a product that reduces the pH of the vagina.

Furthermore, the product is a product that metabolizes in the vaginal environment to produce H₂O₂, organic acids and/or bacteriocins.

Use of the *Lactiplantibacillus plantarum* 1-D1 strain provided by the present application in the preparation of products for regulating the microecological balance of flora.

Use of the *Lactiplantibacillus plantarum* 1-D1 strain provided by the present application in the production of sanitary products.

The present application also protects the use of the *Lactiplantibacillus plantarum* 1-D1 strain as a probiotic.

The present application also provides a product, the active ingredient of which contains *Lactiplantibacillus plantarum,* and the *Lactiplantibacillus plantarum* is the aforementioned *Lactiplantibacillus plantarum* 1-D1 strain.

Furthermore, the product includes but is not limited to food, health products, medicines, daily necessities, etc..

Furthermore, the product is a product for preventing and/or treating vaginal infectious diseases.

The product of the present application may also include a pharmaceutically acceptable excipient or diluent.

Beneficial effects:
(1) The *Lactiplantibacillus plantarum* 1-D1 strain of the present application has genetic stability, no pathogenic/virulence genes, good safety, and exhibits good inhibition ability against *Gardnerella vaginalis, Staphylococcus aureus, Pseudomonas aeruginosa, Escherichia coli, Salmonella paratyphi B* and *Shigella dysenteriae.*
(2) The *Lactiplantibacillus plantarum* 1-D1 strain provided by the present application has a strong ability to produce lactic acid, can reduce the pH in vagina, maintain a healthy vaginal acidic environment, and improve the balance of vaginal flora. At the same time, the antibacterial substances produced by the *Lactiplantibacillus plantarum* 1-D1 strain, such as hydrogen peroxide, bacteriocins, organic acids, etc., can inhibit the growth and reproduction of pathogenic bacteria, improve mucosal immunity, and enhance the anti-infection effect of host.
(3) The *Lactiplantibacillus plantarum* 1-D1 strain provided by the present application has superior cell adhesion, which enables it to adhere to vaginal epithelial cells, forming a microecological barrier to prevent pathogens from colonizing or competing for epithelial cell receptors.
(4) The various evaluation indicators of the *Lactiplantibacillus plantarum* 1-D1 strain provided by the present application are significantly better than commercially available products, such as Wanze Shuangqi and Metronidazole, and have good industrial value and promotion value.

Strain deposit information:
The *Lactiplantibacillus plantarum* 1-D1 strain has been deposited in the China Center for Type Culture Collection on July 28, 2022, with the accession number: CCTCC NO: M 20221191, address: Wuhan, China.

### DESCRIPTION OF THE DRAWINGS

Figure 1 is a frontal photo of the colony morphology of the *Lactiplantibacillus plantarum* 1-D1 strain.
Figure 2 is a Gram staining observation picture of the *Lactiplantibacillus plantarum* 1-D1 strain.
Figure 3 is an ANI comparison of the whole genome between the *Lactiplantibacillus plantarum* 1-D1 stran and the standard strain.
Figure 4 is the result of hemolytic test (left: *Staphylococcus aureus* ATCC 25923 strain; right: *Lactiplantibacillus plantarum* 1-D strain).
Figure 5 is a graph showing the weight change trend of mice in a toxicity test.

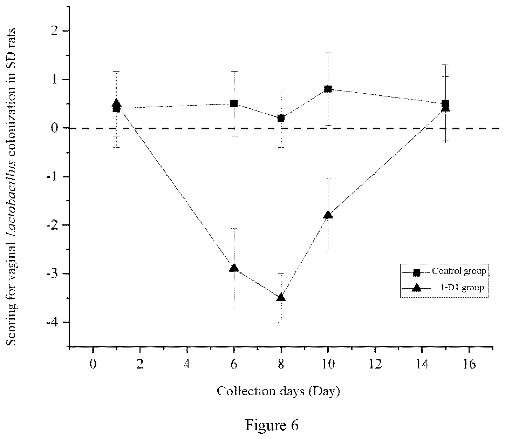

Figure 6 is the score of vaginal *lactobacillus* colonization in SD rats.

### DETAILED DESCRIPTION

In order to enable those skilled in the art to better understand the present application, the present application is further described in detail below in combination with specific examples. Those skilled in the art should understand that this should not be construed as limiting the scope of the claims of the present application. Unless otherwise defined, the technical and scientific terms used in the following examples have the same meaning as those commonly understood by those skilled in the art to which the present application belongs. At the same time, it should also be noted that the reagents or instruments in the present application can be purchased commercially unless otherwise specified. The methods for preparing the culture medium used in the following examples are all well-known methods.

The present application uses the *Lactobacillus delbrueckii* (hereinafter referred to as DJS-H3) isolated from Dingjunsheng as a positive control at each stage, and quickly isolates *lactobacilli* with tolerance to low pH, strong reproductive ability and strong lactic acid production ability through low pH culture medium screening, amplification growth ability and calcium dissolution circle screening. Then, the dominant *lactobacilli* with strong colonization ability and strong ability to inhibit the growth of pathogenic bacteria are screened by the ability to adhere to Hela cells and antibacterial tests. Further in-depth study is conducted on the *Lactiplantibacillus* plantarum 1-D1 strain provided by the present application to investigate its genetic stability and safety, so as to provide further support for its commercial application. Wherein, the representative specific examples are as follows:
DMEM culture medium: Gibco; cat:11995065
MRS liquid culture medium: Huankai Biology, cat:1110151
2% calcium carbonate-0.8% MRS: Sangong, H122BA0030
Agar: Sangong Biotechnology, A505255-0250
HeLa cells: BeNa Culture Collection, catalog number: BNCC342189
VK2/E6E7 cells: Qingqi (Shanghai) Biotechnology

### Example 1 Isolation of Lactobacilli

The vaginal secretions of healthy female volunteers of childbearing age were examined by Gram staining microscopy and subjected to Nugent scoring, and 29 healthy female volunteers of childbearing age were selected. Vaginal cotton swabs from healthy volunteers were placed in MRS acidic liquid culture medium and cultured overnight at 37°C to enrich the bacteria. The enrichment solution was diluted in a 10-fold gradient, and then the diluent at appropriate gradient was spread on 2% calcium carbonate-0.8% MRS agar culture medium and cultured at 37°C for 36-48 hours. Single colonies with obvious transparent circles on the cultured calcium carbonate-MRS culture medium were picked and placed in MRS liquid culture medium, cultured overnight at 37°C, then the cultured 1816 strains were amplified by PCR and sent for 16S rRNA sequencing. After eliminating the remaining acid-producing non-*Lactobacilli,* a total of 72 strains of *Lactobacilli* from different samples were selected for subsequent screening tests.

Among the isolated strains, the nucleotide sequences (16S rRNA sequences) of the four strains of *Lactiplantibacillus plantarum* 1-D1, *Lactobacillus paragasseri* 16-B12, *Lactobacillus jensenii* 53-D2, and *Lactobacillus crispatus* 51S-H2 are shown in SEQ ID No. 1 to 4 respectively.

Preliminary results show that the calcium dissolution circle of the *Lactiplantibacillus plantarum* 1-D1 strain on the calcium carbonate-MRS culture medium was much larger than that of *Lactobacillus delbrueckii* DJS-H3 strain isolated from Dingjunsheng, proving that the *Lactiplantibacillus plantarum* 1-D1 strain has a significant advantage in lactic acid production capacity. The *Lactiplantibacillus plantarum* 1-D1 strain was subjected to 16S rRNA sequencing.

Strain deposit information:
The *Lactiplantibacillus plantarum* strain 1-D1 was deposited in the China Center for Type Culture Collection on July 28, 2022, with the accession number: CCTCC NO: M 20221191, address: Wuhan, China;
The *Lactobacillus crispatus* 51S-H2 strain was deposited in the China Center for Type Culture Collection on July 28, 2022, with the accession number: CCTCC NO: M 20221194, address: Wuhan, China.
The *Lactobacillus paragasseri* 16-B12 strain was deposited in the China Center for Type Culture Collection on July 28, 2022, with the accession number: CCTCC NO: M 20221193, address: Wuhan, China.
The *Lactobacillus jensenii* 53-D2 strain was deposited in the China Center for Type Culture Collection on July 28, 2022, with the accession number: CCTCC NO: M 20221192, address: Wuhan, China.
The *Lactobacillus delbrueckii* (DJS-H3) strain was isolated from Dingjunsheng according to the above method for isolating *Lactiplantibacillus plantarum.*

### Example 2 Determination of growth performance of the Lactiplantibacillus plantarum 1-D1 strain

The growth curve data of *Lactobacillus* at the isolation stage showed that most *Lactobacilli* entered the late logarithmic phase at 12 hours. Therefore, the viable count of each strain was determined by the agar pouring method at 12 hours of inoculation. At the same inoculation time, the strains with high viable counts have a relatively obvious advantage in growth performance. The top 25 strains with growth advantages were selected to enter the next stage of the test. The data showed that the *Lactiplantibacillus plantarum* 1-D1 strain had excellent growth performance and strong reproductive ability, with the viable count reaching 6.37×10⁹ CFU/ml at 12 hours, which was conducive to high-density fermentation during commercial transformation.

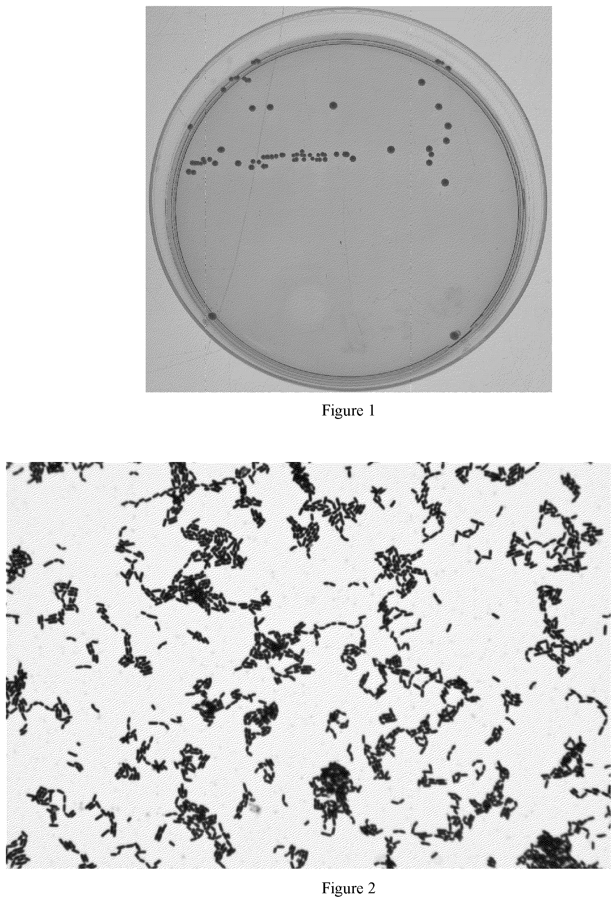

The culture morphology and microscopic characteristics of the *Lactiplantibacillus plantarum* 1-D1 strain were identified, and the results are shown in Figures 1 and 2.

### Example 3 Adhesion ability of the Lactiplantibacillus plantarum 1-D1 strain

The colonization ability of *lactobacilli* was evaluated by the ability of *lactobacilli* to adhere to Hela cells and VK2/E6E7 cells. Firstly, activating *lactobacilli*: 50 µL of each *lactobacillus* was inoculated into 5 ml MRS liquid culture medium, cultured at 37°C for 18-24 hours, and then taken out for transfer. Transfer *Lactobacillus*: 50 µL of each *lactobacillus* activation solution was inoculated into 5 ml MRS liquid culture medium, cultured at 37°C for 18-24 hours, centrifuged at 12000 rpm for 2 minutes, then taken out, resuspended in DMEM culture medium, and adjusted to 0.5 McFarland turbidity. Performing cell plating: 500 µl of the recovered cells with a concentration of 10⁵ cells/mL were inoculated into a 24-well culture plate and placed in a carbon dioxide incubator, and the cells were cultured overnight at 37°C and 5% CO₂. The DMEM stock solution was discarded, the cells were washed 3 times with PBS, and then 500 µL DMEM culture medium was added. Finally, interacting bacteria with cells: 500 µL of *lactobacilli* activated and cultured overnight and resuspended in DMEM culture medium with a concentration of 10⁸ CFU/mL was added to the above 24-well culture plate containing cells, the cells were placed in a carbon dioxide incubator, incubated at 37°C and 5% CO₂ for 2 hours and 4 hours respectively, and then washed 4 times with PBS to remove the non-adherent *lactobacilli.* 500 µL of 0.25% trypsin was added to digest for 2 min, 600 µL of complete culture medium was added to terminate digestion and mixed by pipetting. The cell-bacteria suspension was diluted with sterile PBS in a 10-fold gradient, and 100 µL of the diluent at appropriate gradient was selected and added to a disposable sterile plate, and 0.8% MRS agar culture medium was poured and mixed evenly, and the plate was inverted after solidification. The inverted plate was cultured at 37°C for 48 hours and then taken out for counting. There were 3 parallel plates in each group, and the average value was taken as the final adhesion number. The more *lactobacilli* there are, the stronger their ability to adhere to cells is.

The ability of the *Lactiplantibacillus plantarum* 1-D1 strain to adhere to cells is shown in Tables 1 and 2 below. Through Fisher LSD mean comparison analysis, it was found that the adhesion ability of the *Lactiplantibacillus plantarum* 1-D1 strain was much greater than that of DJS-H3 strain isolated from Dingjunsheng. The differences in adhesion numbers between the groups were extremely significant, the 4 hours adhesion number of the *Lactiplantibacillus plantarum* 1-D1 strain to Hela cells was more than 100 times that of Dingjunsheng, and the 4 hours adhesion number to VK2/E6E7 cells was more than 10 times that of Dingjunsheng.

**Table 1 Adhesion ability of the Lactiplantibacillus plantarum 1-D1 strain to Hela cells at different times**

| Strain number | 2 hours Adhesion number | 4 hours Adhesion number |
|---|---|---|
| 1-D1 | 83.17±1.17^{a} | 132.17±5.17^{a} |
| DJS-H3 | 1±0^{b} | 1.5±0.5^{b} |

| | | |
|---|---|---|
| Note: Different letters indicate significant differences, a: P ≤ 0.05; b: P ≤ 0.01. | | |

**Table 2 Adhesion ability of the Lactiplantibacillus plantarum 1-D1 strain to VK2/E6E7 cells at different times**

| Strain number | 2 hours Adhesion number of single cells | 4 hours Adhesion number of single cells | P value |
|---|---|---|---|
| 1-D1 | 11.46±0.75 | 34.32±3.69 | P≤0.01 |
| DJS-H3 | 3.342±0.23 | 3.57±0.13 | |

### Example 4 Antibacterial ability of the Lactiplantibacillus plantarum 1-D1 strain

Antibacterial tests were used to evaluate the ability of *Lactiplantibacillus plantarum* to inhibit different pathogenic bacteria.

*Lactobacillus* was mixed with 0.8% MRS agar culture medium, and then a 10 mm *lactobacillus* cake was prepared. Different pathogenic bacteria (*Gardnerella vaginalis, Staphylococcus aureus,* etc.) were activated and transferred using culture media and growth conditions suitable for their growth respectively, and then mixed with the corresponding semi-solid culture medium to prepare pathogenic bacteria plates respectively. The *lactobacillus* cake was gently placed on the surface of the pathogenic bacteria plate, incubated upright at 37°C for 18-24 hours, and then taken out, the size of the inhibition zone was measured with a vernier caliper to evaluate the ability of each *lactobacillus* to inhibit pathogenic bacteria. The specific steps were as follows:
After activating *Lactiplantibacillus plantarum* and *Lactobacillus delbrueckii* (DJS-H3), 0.1 ml of bacterial solution was mixed with MRS solid culture medium and poured into a 6 cm plate respectively. After complete solidification, the plate was cultured at 37 °C for 48 hours, then taken out, and holes were punched on the agar culture medium using a puncher with an iner diameter of 10 mm to obtain *lactobacillus* cakes for use.

*Gardnerella vaginalis* and *Atopobium vaginalis* were inoculated into 5% horse serum-anaerobic BHI liquid respectively. After activation and transfer, 0.9% anaerobic saline was diluted 10 times, 1 ml of the diluent was mixed with 10 ml of anaerobic BHI agar containing 5% horse serum, poured into a 9 cm plate, and waited for complete solidification for use.

*Staphylococcus aureus, Pseudomonas aeruginosa, Escherichia coli, Salmonella paratyphi B* and *Shigella dysenteriae* were inoculated into nutrient broth medium respectively. After activation and transfer, the pathogenic bacteria solution was diluted to 100 times with 0.9% saline in a 10-fold gradient. 1 ml of the diluent was mixed with 10 ml of nutrient agar and poured into a 9 cm plate, and waited for complete solidification for use.

The *lactobacillus* cakes were gently placed on the surface of each pathogenic bacteria plate, four cakes were placed symmetrically on each plate, and three parallel plates for each strain. The plates of *Gardnerella vaginalis* and *Atopobium vaginalis* were placed upright in an anaerobic sealed box with an anaerobic gas-producing bag and cultured at 37°C for 48 hours. The plates of other pathogenic bacteria were placed upright in an incubator and cultured at 37°C for 24 hours. Finally, the size of the inhibition zone of each plate was measured with a vernier caliper. The antibacterial ability of different lactobacilli is shown in Table 3.

**Table 3 Size (mm) of inhibition zone of Lactiplantibacillus plantarum against different pathogenic bacteria**

| Strain number | *Staphyloco ccus aureus* | *Escherichi a coli* | *Pseudomo nas aeruginosa* | *Shigella dysenteria e* | *Salmonella paratyphi B* | *Gardnerella vaginalis* | *Atopobi um vaginali s* |
|---|---|---|---|---|---|---|---|
| 1-D1 | 16.67±0.94 | 16.67±0.47 | 27.33±0.47 | 20.67±0.94 | 17.33±1.25 | 17.33±0.47 | 17.27 |
| DJS-H3 | 14.67±0.47 | 14.33±0.47 | 24.67±0.47 | 22±0 | 14.33±0.47 | 15.00±0 | 14.33 |
| Metronid azole (5 µg) | 10.00±0 | 10.00±0 | 10.00±0 | 10.00±0 | 10.00±0 | 12.33±0.47 | 12.99 |

The results showed that the comprehensive antibacterial ability of the *Lactiplantibacillus plantarum* 1-D1 strain was much greater than that of *Lactobacillus delbrueckii* DJS-H3 strain isolated from Dingjunsheng and metronidazole.

### Example 5 Whole genome sequence analysis of the Lactiplantibacillus plantarum 1-D1 strain

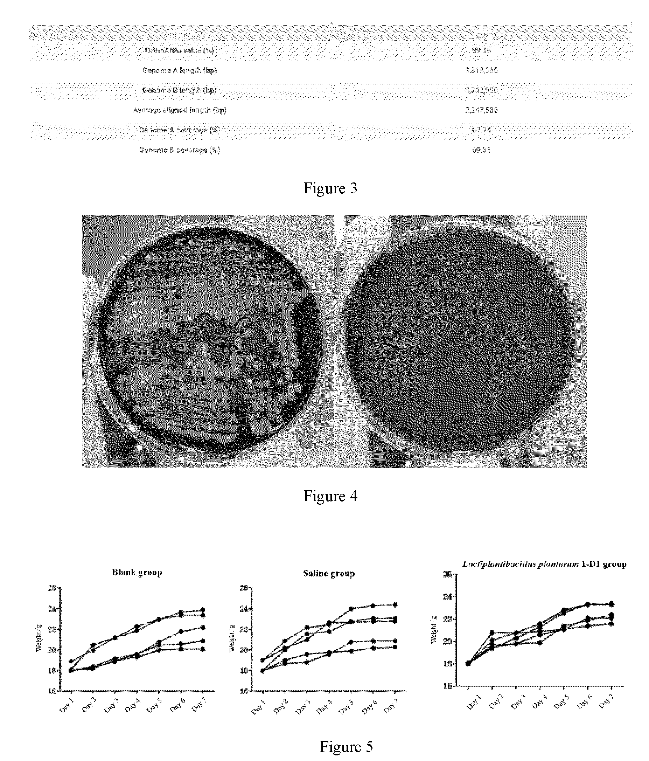

Whole genome sequencing was performed on the *Lactiplantibacillus plantarum* 1-D1 strain. The whole genome sequence of the *Lactiplantibacillus plantarum* 1-D1 strain was uploaded to EzBiocloud, then compared with its standard strain, and the average nucleotide identity (ANI) ratio was obtained as shown in Figure 3. After whole genome sequencing, it was confirmed to be *Lactiplantibacillus plantarum;* Chinese name: 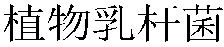 (*Lactiplantibacillus plantarum*).

The virulence genes in the whole genome sequence were analyzed using the Virulence factors Database (VFDB) (identity>70%, coverage length >70%) (A defined commensal consortium elicits CD8 T cells and anti-cancer immunity[J]. Nature, 2019, 565(7741):1), and artificial Blast was performed through the NCBI database, and it was found that two hemolysis-related genes (Hemolysin and Hemolysin III) had 100% identity in *Lactiplantibacillus plantarum.* However, the Hemolysin III gene has been confirmed to be widely present in *Lactiplantibacillus plantarum* (including commercial *Lactiplantibacillus plantarum*: *L. plantarum* 299 V, *L. plantarum* JDM1 and *L. plantarum* ST-III, etc.) (Chokesajjawatee N, Santiyanont P, Chantarasakha K, et al. Safety Assessment of a Nham Starter Culture Lactobacillus plantarum BCC9546 via Whole-genome Analysis[J]. Scientific Reports, 2020, 10(1)), and this gene can only cause lysis of bacteria or fungi, and has not been found to cause lysis in mammalian cells. Therefore, it is believed that strains carrying the Hemolysin III gene will not cause safety issues, while Hemolysin requires the simultaneous presence of multiple genes to exhibit hemolysis (Karina, Arellano, Jorge, et al. Safety Evaluation and Whole-Genome Annotation of Lactobacillus plantarum Strains from Different Sources with Special Focus on Isolates from Green Tea.[J]. Probiotics and antimicrobial proteins, 2019), and 1-D1 does not contain genes that interact with Hemolysin to cause hemolysis. At the same time, the *Lactiplantibacillus plantarum* 1-D1 strain has no plasmid, and the transferable elements in the genome do not contain resistance genes, so there is no risk of resistance gene transfer. In summary, the *Lactiplantibacillus plantarum* 1-D1 strain has good safety at the genetic level.

### Example 6 Hemolytic test of the Lactiplantibacillus plantarum 1-D1 strain

In order to further confirm whether the *Lactiplantibacillus plantarum* 1-D1 strain is hemolytic, the *Lactiplantibacillus plantarum* 1-D1 strain was streaked on a blood agar plate (Chengdu Ruiqi; Sichuan Medical Device Registration Approval No. 20152400001) and cultured anaerobically for 48 hours, and the *Staphylococcus aureus* ATCC 25923 strain (Shanghai Bioplus Biotech) with β-hemolysis was used as a positive control and cultured aerobically for 24 hours. Whether the *Lactiplantibacillus plantarum* 1-D1 strain is hemolytic can be determined by observing whether a hemolytic ring is formed by the *Lactiplantibacillus plantarum* 1-D1 strain on the blood agar plate after cultivation.

The results in Figure 4 show that obvious transparent circles can be seen around the colonies of ATCC 25923 of the positive control plate, showing typical β-hemolytic characteristics, and the *Lactiplantibacillus plantarum* 1-D1 strain does not produce any hemolytic circle, indicating that the *Lactiplantibacillus plantarum* 1-D1 strain does not produce hemolysin to dissolve red blood cells, that is, it is not hemolytic.

### Example 7 Toxicity test of the Lactiplantibacillus plantarum 1-D1 strain in mice

Five mice weighing 18-22 g were used, and each mouse was orally gavaged with 0.5 ml of fresh bacterial solution of the *Lactiplantibacillus plantarum* 1-D1 strain (not less than 1.0×10⁹ CFU/0.5 ml) once a day for 3 consecutive days, and the mice were observed continuously from the first day of gavage to the seventh day.

The toxicity test in mice have shown that the mice were observed to have normal body posture, normal limb movement, flexible movement, smooth fur color, normal eating and drinking, no abnormal excretion and secretion were found, no obvious symptoms of poisoning was found, no animals died abnormally during the experiment, and the mice all gained weight.

### Example 8 Antibiotic sensitivity test of the Lactiplantibacillus plantarum 1-D1 strain

According to the requirements of antibiotic sensitivity test in the general introduction of microecological live bacteria products in Part III of the 2020 edition of the Pharmacopoeia, the Agar Kirby-Bauer test was used to determine the sensitivity of the strain to antibiotics, and the antibiotic sensitivity level of the strain was determined according to the size of the inhibition zone.

The standard sensitive strains *Escherichia coli* ATCC 25922 and *Staphylococcus aureus* ATCC 25923 were used as quality control bacteria. The analysis was carried out in accordance with the international standards of the Clinical and Laboratory Standards Institute (CLSI) of the United States and the health industry standards of the People's Republic of China. The sensitivity judgment standard of the *Lactiplantibacillus plantarum* 1-D1 strain was formulated based on the judgment standards of quality control strains. The sensitivity levels was divided into sensitive, intermediate and resistant, which were represented by S, I and R respectively.

The results of antibiotic sensitivity test showed that the *Lactiplantibacillus plantarum* 1-D1 strain was resistant to penicillin, oxacillin, azithromycin, gentamicin, kanamycin, vancomycin, enrofloxacin, clindamycin and metronidazole, sensitive to ampicillin, imipenem, ceftriaxone, amoxicillin, piperacillin, tetracycline, and moderately sensitive to erythromycin. On the other hand, metronidazole and clindamycin are commonly used in clinical treatment of bacterial vaginosis, and the results of this experiment suggest that the *Lactiplantibacillus plantarum* 1-D1 strain can theoretically be used in combination with metronidazole and clindamycin.

**Table 4 Antibiotic types, drug content of drug-sensitive paper strips and criteria for judging the diameter of the inhibition zone**

| Antibiotic type | Antibiotic name | Abbr. | Specification | Criteria for judging the diameter of the inhibition zone of *Lactiplantibacillus plantarum* | | | *Escherichia coli* ATCC 25922 | | | *Staphylococcus aureus* ATCC 25923 | | |
|---|---|---|---|---|---|---|---|---|---|---|---|---|
| | | | | S | I | R | S | I | R | S | I | R |
| β-lactams | Ampicillin | AMP | 10 µg/tablet | ≥17 | 14-16 | ≤13 | ≥17 | 14-16 | ≤13 | ≥29 | - | ≤28 |
| | Imipenem | IMP | 10 µg/tablet | ≥16 | 14-15 | ≤13 | ≥23 | 20-22 | ≤19 | ≥16 | 14-15 | ≤13 |
| | Ceftriaxone | CTR | 30 µg/tablet | ≥21 | 14-20 | ≤13 | ≥23 | 20-22 | ≤19 | ≥21 | 14-20 | ≤13 |
| | Penicillin | PEN | 10 U/tablet | ≥29 | - | ≤28 | / | / | / | ≥29 | - | ≤28 |
| | Amoxicillin | AMX | 25 µg/tablet | ≥18 | 14-17 | ≤13 | ≥18 | 14-17 | ≤13 | ≥20 | - | ≤19 |
| | Oxacillin | OXA | 1 µg/tablet | ≥13 | 11-12 | ≤10 | / | / | / | ≥13 | 11-12 | ≤10 |
| | Piperacillin | PIP | 10 µg/tablet | ≥21 | 18-20 | ≤17 | ≥21 | 18-20 | ≤17 | ≥18 | - | ≤17 |
| Macrolides | Erythromycin | E | 15 µg/tablet | ≥23 | 14-22 | ≤13 | / | / | / | ≥23 | 14-22 | ≤13 |
| | Azithromycin | AZM | 15 µg/tablet | ≥13 | - | ≤12 | ≥13 | - | ≤12 | ≥18 | 14-17 | ≤13 |
| Aminoglycosides | Gentamycin | GM | 120 µg/tablet | ≥15 | 13-14 | ≤12 | ≥15 | 13-14 | ≤12 | ≥15 | 13-14 | ≤12 |
| | Kanamycin | K | 30 µg/tablet | ≥18 | 14-17 | ≤13 | ≥18 | 14-17 | ≤13 | ≥18 | 14-17 | ≤13 |
| Tetracyclines | Tetracycline | TET | 30 µg/tablet | ≥15 | 12-14 | ≤11 | ≥15 | 12-14 | ≤11 | ≥19 | 15-18 | ≤14 |
| Glycopeptides | Vancomycin | VAN | 30 µg/tablet | ≥15 | - | - | / | / | / | ≥15 | - | - |
| Quinolones | Enrofloxacin | ENR | 5 µg/tablet | ≥21 | 16-20 | ≥15 | ≥21 | 16-20 | ≤15 | ≥21 | 16-20 | ≤15 |
| Lincosamides | Clindamycin | CC | 2 µg/tablet | ≥21 | 15-20 | ≤14 | / | / | / | ≥21 | 15-20 | ≤14 |
| Nitroimidazoles | Metronidazole | MTZ | 5 µg/tablet | / | / | / | / | / | / | / | / | / |
| | | | 50 µg/tablet | | | | | | | | | |

| | | | | | | | | | | | | |
|---|---|---|---|---|---|---|---|---|---|---|---|---|
| Notes: ① "-" indicates no inhibition zone or the drug sensitivity K-B test is not applicable, and "/" indicates no reference standard. ② The sensitivity judgment standards for *Escherichia coli* ATCC 25922 and *Staphylococcus aureus* ATCC 25923 and the allowable range of the inhibition zone diameter of quality control strains are derived from the international standards of the Clinical and Laboratory Standards Institute (CLSI) of the United States and the health industry standards of the People's Republic of China. | | | | | | | | | | | | |

**Table 5 Inhibition zone diameter and judgement results of drug sensitivity test of the Lactiplantibacillus plantarum 1-D1 strain**

| Antibioti c type | Antibiotic | Specificatio n | *Lactiplantibacillus plantarum* 1-D1 | | Quality control strains | | | |
|---|---|---|---|---|---|---|---|---|
| | | | | | *Escherichia coli* ATCC 25922 | | *Staphylococcus aureus* ATCC 25923 | |
| | | | Diameter of inhibition zone (mm) | Judgement result | Diameter of inhibition zone (mm) | Judgement result | Diameter of inhibition zone (mm) | Judgement result |
| β-lactam s | Ampicillin | 10 µg/tablet | 27 | S | 15 | I | 27 | R |
| | Imipenem | 10 µg/tablet | 30 | S | 27 | S | 41 | S |
| | Ceftriaxone | 30 µg/tablet | 25 | S | 24 | S | 22 | S |
| | Penicillin | 10 U/tablet | 28 | R | 6 | R | 30 | S |
| | Amoxicilli n | 25 µg/tablet | 30 | S | 18 | S | 33 | S |
| | Oxacillin | 1 µg/tablet | 6 | R | 6 | R | 10 | R |
| | Piperacillin | 10 µg/tablet | 30 | S | 18 | I | 26 | S |
| Macrolid es | Erythromyc in | 15 µg/tablet | 21 | I | 6 | R | 24 | S |
| | Azithromyc in | 15 µg/tablet | 11 | R | 28 | S | 22 | S |
| Aminogl ycosides | Gentamyci n | 120 µg/tablet | 11 | R | 21 | S | 25 | S |
| | Kanamycin | 30 µg/tablet | 6 | R | 17 | I | 20 | S |
| Tetracyc lines | Tetracyclin e | 30 µg/tablet | 20 | S | 18 | S | 24 | S |
| Glycope prides | Vancomyci n | 30 µg/tablet | 6 | R | 6 | R | 17 | S |
| Quinolo nes | Enrofloxaci n | 5 µg/tablet | 6 | R | 25 | S | 29 | S |
| Lincosa mides | Clindamyci n | 2 µg/tablet | 11 | R | 6 | R | 24 | S |
| Nitroimi dazoles | Metronidaz ole | 5 µg/tablet | 6 | R | 6 | R | 6 | R |
| | | 50 µg/tablet | 6 | R | 6 | R | 6 | R |

### Example 9 Colonization test of the Lactiplantibacillus plantarum 1-D1 strain in rat vagina

SD rats were given the *Lactiplantibacillus* plantarum 1-D1 strain once a day through vaginal administration for 5 consecutive days and then observed for 10 days. Vaginal secretions were collected before each administration and subjected to Gram staining microscopy (secretions on D1 were collected before the initial administration) to determine the colonization of the *Lactiplantibacillus* plantarum 1-D1 strain. The experimental scheme is shown in Table 6 below.

Gram staining microscopy showed that there were no microorganisms in the vaginal secretions of most rats before the initial administration, and *Gram-negative bacilli* and *Gram-positive cocci* were occasionally found in the vaginal secretions of a very small number of rats. After continuous administration, compared with the blank control group, the rats given the *Lactiplantibacillus plantarum* 1-D1 strain had significantly more *Gram-positive bacilli* in their vaginal secretions than before administration and the blank control group, indicating that the *Lactiplantibacillus plantarum* 1-D1 strain can successfully colonize in the vagina of SD rats. Five days after stopping the administration (D10), *Gram-positive bacilli* were still visible in the field of vision. Ten days after stopping the administration (D15), the vaginal microenvironment of the rats returned to the state before administration.

**Table 6 Experimental scheme of colonization of the Lactiplantibacillus plantarum 1-D1 strain in rat vagina**

| Group | Number of animals | Dosage | Administration time | Observation period | Collection time of vaginal secretions | Detection index |
|---|---|---|---|---|---|---|
| | | | D1-D5 | D6-D15 | | |
| Blank excipient control group | 10 | 0 | D1-D5 | D6-D15 | D1, D6, D8, D10 and D15 | Gram staining microscopy |
| *Lactiplantibacillus plantarum* 1-D1 group | 10 | 1×10⁸ CFU | D1-D5 | D6-D15 | | |

**Table 7 Scoring criteria for vaginal Lactobacillus colonization in SD rats**

| Score | | Number of bacteria under the field of vision of microscopic examination |
|---|---|---|
| Score of *Gram-positive bacilli (Lactobacillus)* | -4 | ++++ (≥30 *Lactobacilli*) |
| | -3 | +++ (5-30 *Lactobacilli*) |
| | -2 | ++ (1-4 *Lactobacillli*) |
| | -1 | + (≥1 *Lactobacillus*) |
| Score of non-*Gram-positive bacilli* | 0 | No colonies |
| | 1 | With *Gram-negative bacteria, Gram-positive cocci,* etc. |

| | | |
|---|---|---|
| Note: A score of <0 indicates successful *Lactobacillus* colonization; a score of ≥0 indicates a failure of *Lactobacillus* colonization. | | |

Those skilled in the art should understand that the embodiments of the present application described above and shown in the accompanying drawings are only examples and are not intended to limit the present application. The objects of the present application has been fully and effectively achieved. The functional and structural principles of the present application have been demonstrated and explained in the embodiments. Without departing from the principles, the embodiments of the present application may be deformed or modified in any way.

## Claims

1. A *Lactiplantibacillus plantarum* 1-D1 strain, wherein, the accession number of the strain is: CCTCC NO: M 20221191.

2. Use of the *Lactiplantibacillus plantarum* 1-D1 strain according to claim 1 in the preparation of an antibacterial product;
preferably, the antibacterial product is a product that inhibits one or more bacteria of *Gardnerella, Staphylococcus aureus, Pseudomonas aeruginosa, Escherichia coli, Salmonella,* and *Shigella dysenteriae.*

3. Use of the *Lactiplantibacillus plantarum* 1-D1 strain according to claim 1 in the preparation of products for preventing and/or treating vaginal infectious diseases.

4. The use according to claim 3, wherein, the vaginal infectious disease is caused by one or more of bacterial vaginal pathogenic bacterium, *vulvovaginal candidiasis* pathogenic bacterium, trichomonas vaginitis, and aerobic vaginitis;
preferably, the pathogenic bacterium of the aerobic vaginitis includes but is not limited to *Staphylococcus aureus, Escherichia coli, Pseudomonas aeruginosa, Shigella dysenteriae* or *Salmonella*;
preferably, the bacterial vaginal pathogenic bacterium is *Gardnerella vaginalis.*

5. The use according to claim 3, wherein, the product is a product that reduces the pH of vagina.

6. The use according to claim 3, wherein, the product is a product that metabolizes in the vaginal environment to produce H₂O₂, organic acids and/or bacteriocins.

7. Use of the *Lactiplantibacillus plantarum* 1-D1 strain according to claim 1 in the preparation of products for regulating the microecological balance of flora.

8. Use of the *Lactiplantibacillus plantarum* 1-D1 strain according to claim 1 in the production of sanitary products.

9. A product, wherein, the active ingredient of the product contains *Lactiplantibacillus plantarum*; the *Lactiplantibacillus plantarum* is the *Lactiplantibacillus plantarum* 1-D1 strain according to claim 1.

10. The product according to claim 9, wherein, the product is a food, a health product, a medicine or a daily necessity;
preferably, the product is a product for preventing and/or treating vaginal infectious diseases.
